# EUROPÄISCHE PATENTSCHRIFT

(11) **EP 0 655 071 B1**
(45) Veröffentlichungstag und Bekanntmachung des Hinweises auf die Patenterteilung: **13.08.1997**
(21) Anmeldenummer: 93914706.2
(22) Anmeldetag: 22.06.1993
(51) Int. Cl.: C07J 1/00, C07J 21/00

(54) **VERFAHREN ZUR HERSTELLUNG VON 1-METHYL-3-KETO-$g(D)?1,4 -STEROIDEN**
METHOD OF PREPARING 1-METHYL-3-KETO-$g(D)?1,4 -STEROIDS
PROCEDE DE FABRICATION DE 1-METHYL-3-CETO-$g(D)?1,4 -STEROIDES

(30) Priorität: 13.08.1992 DE 4227053
(43) Veröffentlichungstag der Anmeldung: 31.05.1995
(73) Patentinhaber: SCHERING AKTIENGESELLSCHAFT, 13342 Berlin (DE)
(72) Erfinder: WESTERMANN, Jürgen, D-12161 Berlin (DE); NICKISCH, Klaus, D-12307 Berlin (DE); PRELLE, Annette, D-12103 Berlin (DE)
(86) Internationale Anmeldenummer: EP9301606
(87) Internationale Veröffentlichungsnummer: WO9404553

(56) Entgegenhaltungen:
- EP-A- 0 290 378
- EP-A- 0 534 582
- AUSTRALIAN JOURNAL OF CHEMISTRY Bd. 28, Nr. 4, 1975, MELBOURNE, AU Seiten 801 - 815 L. BAGNELL ET AL 'Organoaluminum Compounds. XXIX. Nickel Catalysed Conjugate Addition of Trimethylaluminum to alpha,beta-Unsaturated Ketones' in der Anmeldung erw hnt s
- AUSTRALIAN JOURNAL OF CHEMISTRY Bd. 28, Nr. 4, 1975, MELBOURNE, AU Seiten 817 - 820 L. BAGNELL ET AL 'Organoaluminum Compounds. XXX. Nickel Catalysed Conjugate Addition of Trimethylaluminum to 3-oxo-delta-4-Steroids' in der Anmeldung erw hnt s
- JOURNAL OF ORGANIC CHEMISTRY. Bd. 39, Nr. 22, 1. November 1974, EASTON US Seiten 3297 - 3299 E. AHSBY ET AL 'Transition Metal Catalyzed Conjugate Methylation of alpha,beta-Unsaturated Ketones by Trimethylaluminum and Lithium Tetramethylaluminate' s

## Beschreibung

Die Erfindung betrifft ein Verfahren zur Herstellung von 1-Methyl-3-keto-Δ^{1,4}-steroiden der allgemeinen Formel I 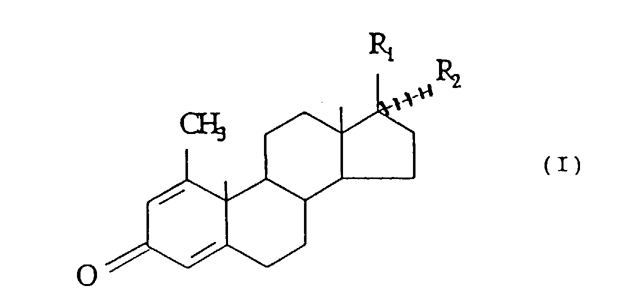 worin R¹ und R² unten definiert werden, dadurch gekennzeichnet, daß man ein 3-Keto-Δ^{1,4}-steroid der allgemeinen Formel II 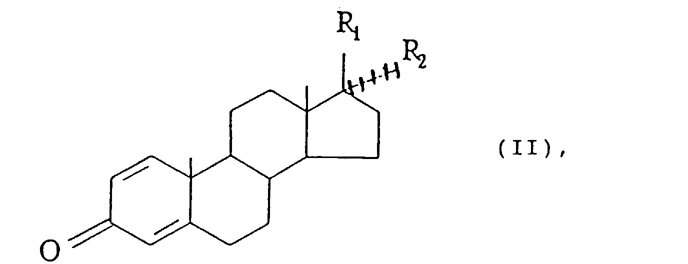 worin R¹ und R² die obengenannten Bedeutungen besitzten in einem inerten Lösungsmittel in Gegenwart eines hierin löslichen Nickelsalzes mit einer metallorganischen Verbindung der Formel III, IV oder V

CH₃ - Zn - X (III),

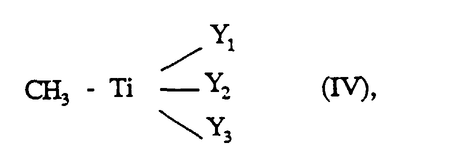

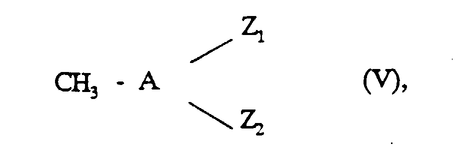

worin
X eine Methylgruppe, eine Alkoxygruppe mit maximal 6 Kohlenstoffatomen oder eine gegebenenfalls durch Halogenatome, Alkoxygruppen mit maximal 6 Kohlenstoffatomen und/oder Alkylgruppen mit maximal 6 Kohlenstoffatomen substituierter Phenoxyrest darstellt,
   und
Y₁, Y₂ und Y₃ sowie Z₁ und Z₂ gleich oder verschieden sind und die selbe Bedeutung wie X besitzen, bei einer Temperatur zwischen 40 bis 80 °C umsetzt.
R₁ und R₂ stellen gemeinsam eine Oxogruppe oder eine Alkylendioxygruppe mit 2 bis 6 Kohlenstoffatomen dar oder
R₁ bedeutet eine Acyloxygruppe mit bis zu 8 Kohlenstoffatomen oder einen Alkylrest mit maximal 10 Kohlenstoffatomen und
R₂ symbolisiert ein Wasserstoffatom.

Unter einer Alkylendioxygruppe R₁ und R₂ soll beispielsweise eine Ethylendioxygruppe, eine 1,3-Propylendioxygruppe, eine 2.2-Dimethylpropylendioxygruppe oder eine 2.3-Butylendioxygruppe verstanden werden.

Unter einer Acyloxygruppe R₁ der Verbindungen soll vorzugsweise eine Alkanoylgruppe wie die Acetoxygruppe, die Propionyloxygruppe, die Butyryloxygruppe, die Trimethylacetoxygruppe, etc. oder die Benzoyloxygruppe verstanden werden.

Unter einem Alkylrest R₁ soll vorzugsweise ein solcher mit 8 bis 10 Kohlenstoffatomen verstanden werden, wie er in den Seitenketten von natürlich vorkommenden Zoo- oder Phytosterinen, wie Cholesterin, Campesterin oder β-Sitosterin vorliegt.

Es ist bekannt, daß das 1-Methyl-androsta-1,4-dien-3,17-dion (=Atamestan) eine pharmakologisch wirksame Verbindung ist, deren mehrstufige Synthese aus Androsta-1,4-dien-3,17-dion aber recht aufwendig ist (DE-A 40 15 247). Demgegenüber ermöglicht es das erfindungsgemäße Verfahren, diese Umsetzung in einem Reaktionsschritt durchzuführen. Das dies möglich ist, ist für den Fachmann überraschend, denn es ist bekannt, das unter vergleichbaren Bedingungen 3-Keto-Δ⁴-steroide in der 5β-Position methyliert werden (Aust. J. Chem. 1975, 28, 817ff). Auch die übrigen Verbindungen der allgemeinen Formel I lassen sich in einfacher Weise in Atamestan überführen, indem man die 17-Ester oder 17-Ketale verseift und anschließend die 17-Hydroxygruppe in bekannter Weise oxydiert (DE-A 33 22 285) oder indem man die Seitenkette der Sterin-Derivate der allgemeinen Formel I unter Bedingungen, wie sie im US-A 4,100,026 beschrieben sind, mikrobiologisch abbaut.

Das erfindungsgemäße Verfahren wird in einem inerten Lösungsmittel durchgeführt. Geeignete Lösungsmittel sind aliphatische, cycloaliphatische oder aromatische flüssige Kohlenwasserstoffe, wie Hexan, Petrolether, Cyclohexan, Benzol, Toluol oder Xylol, Ether, wie Diethylether, Diisopropylether, Tetrahydrofuran oder Dioxan, Ester wie Essigsäureethylester, Essigsäuremethylester oder Gemische dieser Lösungsmittel.

Die Reaktion wird in Gegenwan von Nickelsalzen durchgeführt, die in diesen Lösungsmitteln oder Lösungsmittelgemischen löslich sind. Geeignete Nickelsalze sind beispielsweise das Nickel(II)-bis-(triphenylphosphin)-chlorid, das Tetrakis-(triphenylphosphin)-nickel(O), das Nickel(II)-stearat, das Bis-(diethyloxalacetatol)-nickel, das Pentakis-(ethylacetatol-hydroxotrinickel, das Bis(1,3-diphenylpropan-1,3-dionatolnickel, das Bis(acrolein)-nickel(O) und insbesondere das Nickel(II)-acetylacetonat (Aust. J. Chem., 1974, 27, 2569 ff und Aust. J. Chem., 1975, 28, 801 ff). Zur Reaktion verwendet man vozugsweise 0,01-0,1 Mol Nickelsalz pro Mol umzusetzendes Steroid. Erfindungsgemäß verwendet man zur Methylierung des Steroids eine metallorganische Verbindung der Formel III, IV oder V. Als eine geeignete metallorganische Verbindung der allgemeinen Formel III sei beispielsweise das Dimethylzink genannt. Eine metallorganische Verbindung der Formel IV ist zum Beispiel das Methyl-triisopropyloxy-titan (J. Organomet. Chem., 1974, 74, 85ff). Bevorzugt erfolgt die Methylierung mit aluminiumorganischen Verbindungen der allgemeinen Formel V, wobei gegenüber dem Trimethylaluminium solche Verbindungen besonders bevorzugt sind, in denen einer oder beide Reste Z₁ und Z₂ eine Alkoxygruppe oder einen gegebenenfalls substituierten Phenoxyrest darstellen. Diese Verbindungen lassen sich in einfacher Weise herstellen, indem man beispielsweise eine Lösung von Trimethylaluminium in einem Kohlenwasserstoffrest mit der stöchiometrisch benötigten Menge eines Alkohols oder eines vorzugsweise sterisch gehinderten Phenols umsetzt. (T. Mole und E. Jeffry in "Organoaluminium Compounds", Elviser Publishing Comp. Amsterdam, London, New York 1972 und J. Org. Chem. 1979, 26, 4792 ff).

Geeignete Alkohole sind beispielsweise Methanol, Ethanol, Isopropanol oder tert.-Butanol, als geeignete Phenole seien beispielsweise genannt, das 2,6-Di-tert.-butylphenol, 2-tert.-Butyl-6-methylphenol, 2,4,6-Tri-tert.-butylphenol, 2,6-Di-tert.-butyl-4-bromphenol oder besonders auch das 2,6-Di-tert.-butyl-4-methylphenol.

Zur Durchführung der Reaktion verwendet man vorzugsweise 0,3 bis 1,2 Mol das 1 bis 1,5-fache der theoretisch benötigten Menge an metallorganischer Verbindung.

Die Reaktion selbst kann beispielsweise so durchgeführt werden, daß man eine Lösung des umzusetzenden Steroids in eine Lösung der metallorganischen Verbindung einträgt, den Nickelkatalysator zusetzt und die Mischung auf 40 °C bis 80 °C erwärmt. Andererseits kann der Nickelkatalysator aber auch mit dem Steroid vorgelegt und die metallorganische Verbindung zudosiert werden.

Nach erfolgter Umsetzung, die beispielsweise durch dünnschichtchromatographische oder gaschromatographische Analyse ermittel werden kann, wird das Reaktionsgemisch in an sich bekannter Weise aufbereitet und das Reaktionsprodukt durch Chromatographie und/oder Umkristallisation aufgereinigt.

Die nachfolgenden Ausführungsbeispiele dienen zur näheren Erläuterung des erfindungsgemäßen Verfahrens.

### Beispiel 1

### 1-Methyl-androsta-1,4-dien-3,17-dion

Bei Raumtemperatur werden 12 ml (12 mmol) Trimethylaluminium einer 10 %igen Lösung in Hexan unter Stickstoffatmosphäre vorgelegt. Unter Rühren werden 2,64 g 2,6-Di-tert-butyl-4-methylphenol portionsweise zugegeben. Die Lösung wird 30 Minuten nachgerührt und bei 25 °C werden 2,84 g (10 mmol) Androsta-1,4-dien-3,17-dion in 20 ml Ethylacetat zugegeben. Die Lösung wird auf 58 °C erwärmt. Bei 58 °C werden 143 mg Nickel(II)-acetylacetonat zugegeben. Die Lösung wird noch 2,5 Stunden bei einer Temperatur von 60 °C gerührt. Nach Abkühlen werden zur Hydrolyse 1,1 ml Wasser zugegeben und 15 Minuten gerührt. Der anorganische Feststoff wird abfiltriert und zweimal mit 50 ml Ethylacetat nachgewaschen. Nach Einengen der Ethylacetatphasen wird ein Rohprodukt erhalten, das an Silicagel mit Hexan/Ethylacetat als Eluens mit steigendem Ethylacetatanteil chromatographiert wird. Es werden 1,5 g Ausgangsmaterial zurückgewonnen. Es werden 0,60 g 1-Methyl-androsta-1,4-dien-3,17-dion vom Schmelzpunkt 168-170 °C erhalten. Die Ausbeute beträgt 20 % der Theorie bzw. 42 % unter Berücksichtigung zurückgewonnenem Ausgangsmaterial.

### Beispiel 2

### 17β-Acetoxy-1-methyl-androsta-1,4-dien-3-on

Bei Raumtemperatur werden 12 ml Trimethylaluminium 10 %ig in Hexan (12 mmol) unter Stickstoffatmosphäre vorgelegt. Unter Rühren werden 2,64 g 2,6-Di-tert-butyl-4-methylphenol portionsweise zugegeben. Die Lösung wird 30 Minuten nachgerührt und bei 25 °C werden 3,27 g (10 mmol) 17β-Acetoxy-androsta-1,4-dien-3-on in 20 ml Ethylacetat zugegeben. Die Lösung wird auf 60 °C erwärmt und bei 60 °C werden 143 mg Nickel(II)-acetylacetonat zugegeben. Die Lösung wird noch 2,5 Stunden bei dieser Temperatur gerührt. Nach Abkühlen werden zur Hydrolyse 1,1 ml Wasser zugegeben und 15 Minuten gerührt. Der Feststoff wird abfiltriert, zweimal mit 50 ml Ethylacetat nachgewaschen. Nach Einengen der Ethylacetatphasen wird ein Rohprodukt erhalten, das an Silicagel mit Hexan/Ethylacetat als Eluens mit steigendem Ethylacetatanteil chromatographiert wird. Es werden 1,55 g Ausgangsmaterial zurückgewonnen. Es werden 0,68 g 17β-Acetoxy-1-methyl-androsta-1,4-dien-3-on vom Schmelzpunkt 144,1 °C erhalten. Die Ausbeute beträgt 20 % der Theorie bzw. 38 % unter Berücksichtigung zurückgewonnenem Ausgangsmaterial.

### Beispiel 3

### 1-Methyl-androsta-1,4-dien-3,17-dion

Bei Raumtemperatur werden 86,4 ml (80 mmol) einer 10%igen Lösung von Trimethylaluminium in Hexan unter Stickstoffatmosphäre vorgelegt. Unter Rühren werden 17,4 g (80 mmol) 2,6-Di-tert-butyl-4-methylphenol portionsweise zugegeben. Die Lösung wird 30 Minuten nachgerührt und bis Ende der Methanentwicklung nachgerührt. Bei 25°C werden 28,4 g (100 mmol) Androsta-1,4-dien-3,17-dion (ADD) in 200 ml Methylacetat zugegeben. Die Lösung wird auf 50°C erwärmt. Bei 50°C werden werden 1,43 g (5 mmol) Nickel(II)-acetylacetonat zugegeben. Die Lösung wird noch 50 Minuten bei einer Temperatur von 50°C nachgerührt. Zur Hydrolyse werden unter Kühlung 15 ml Wasser zugegeben, zur Vervollständigung wird noch 15 Minuten nachgerührt. Der anorganische Feststoff wird abfiltriert und zweimal mit 50 ml Methylacetat nachgewaschen. Nach Einengen der Methylacetatphasen weden 43,71 g Rohprodukt erhalten, das an Silicagel mit Hexan/Ethylacetat als Eluens mit steigendem Ethylacetatanteil chromatographiert wird. Nach Einengen der Fraktionen werden 6,5 g 1-Methyl-androsta1,4-dien-3,17-dion (23% der Theorie) vom Schmelzpunkt 168°C erhalten.

### Beispiel 4

### a) 1-Methyl-androsta-1,4-dien-3,17-dion-17-ethylenketal

Bei Raumtemperatur werden 32,22 (mmol) einer 10%igen Lösung von Trimethylaluminium in Hexan unter Stickstoffatmosphäre vorgelegt. Unter Rühren werden 6,6 g (30 mmol) 2,6-Di-tert-butyl-4-methylphenol portionsweise zugegeben. Die Lösung wird 30 Minuten bei 35°C nachgerührt. Diese so hergestellte Lösung von Dimethylaluminium-2,6-di-tert-butyl-4-methylphenoxid wird bei 58°C zu einer Lösung aus 9,84 g (30 mmol) Androsta-1,4-dien-3,17-dion-17-ethylenketal und 430 mg (1,5 mmol) Nickel(II)-acetylacetonat in 60 ml Ethylacetat (Essigsäure-ethylester) gegeben. Die Lösung wird noch 4 Stunden bei einer Temperatur von 60°C gerührt. Zur Hydrolyse werden unter Kühlung 5 ml Wasser zugegeben. Der anorganische Feststoff wird abfiltriert und zweimal mit 20 ml Ethylacetat nachgewaschen. Nach Einengen der Ethylacetatphasen werden 17 g Rohmaterial erhalten, das an Silicagel mit Hexan/Ethylacetat als Eluens mit steigendem Ethylacetatanteil chromatographiert wird. Nach Einengen der Fraktionen werden 12,9 g 1-Methyl-androsta-1,4-dien-3,17-dion-17-ethylenketal (28 % der Theorie) vom Schmelzpunkt 159°C erhalten.

### b) 1-Methyl-androsta-1,4-dien-3,17-dion

3,42 g (10 mmol) 1-Methyl-androsta-1,4-dien-3,17-dion-17-ethylen-ketal aus Beispiel 4a werden in 20 ml Ethanol gelöst. Es werden 3 ml 2 n Schwefelsäure zugegeben und die Lösung 4 Stunden bei Raumtemperatur gerührt. Das Reaktionsgemisch wird auf 60 ml Eiswasser gegeen und abfiltriert. Kristallisation des Niederschlages aus 10 ml Ethylacetat ergibt 2,65 g 1-Methyl-androsta-1,4-dien-3,17-dion (88% der Theorie) vom Schmelzpunkt 170°C.

## Patentansprüche

1. Verfahren zur Herstellung von 1-Methyl-3-keto-Δ^{1,4}-steroiden der allgemeinen Formel I 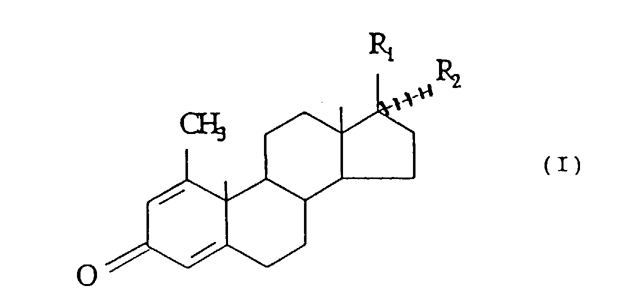 worin
R₁ und R₂ gemeinsam eine Oxogruppe oder eine Alkylendioxygruppe mit 2 bis 6 Kohlenstoffatomen darstellen oder worin
R₁ eine Acyloxygruppe mit bis zu 8 Kohlenstoffatomen oder einen Alkylrest mit maximal 10 Kohlenstoffatomen bedeutet und
R₂ ein Wasserstoffatom symbolisiert,
dadurch gekennezeichnet, daß man aus 3-Keto-Δ^{1,4}-steroiden der allgemeinen Formel II 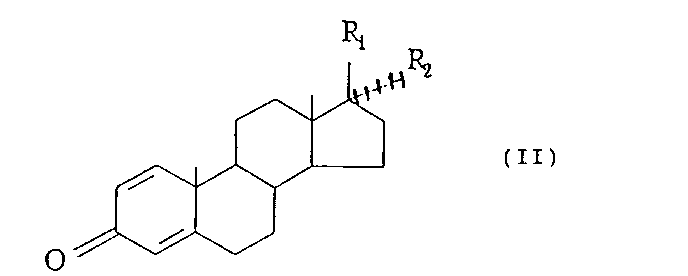 worin R₁ und R₂ die obengenannten Bedeutungen besitzen, in einem inerten Lösungsmittel in Gegenwart eines hierin löslichen Nickelsalzes mit einer metallorganischen Verbindung der Formel III, IV oder V
CH₃ - Zn - X (III), 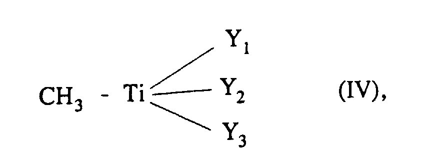
oder 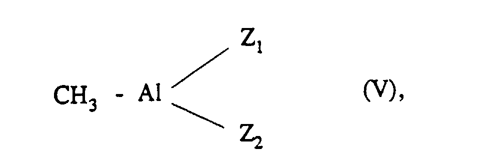 worin
X eine Methylgruppe, eine Alkoxygruppe mit maximal 6 Kohlenstoffatomen oder eine gegebenenfalls durch Halogenatome, Alkoxygruppen mit maximal 6 Kohlenstoffatomen und/oder Alkylgruppen mit maximal 6 Kohlenstoffatomen substituierter Phenoxyrest darstellt,
und
Y₁, Y₂ und Y₃ sowie Z₁ und Z₂ gleich oder verschieden sind und dieselbe Bedeutung wie X besitzen, bei einer Temperatur zwischen 40°C bis 80°C in einem Reaktionsschritt umsetzt, wobei die 1-Methylgruppe bei gleichzeitiger Rückbildung der Doppelbindung eingeführt wird.

## Claims

1. Process for the manufacture of 1-methyl-3-keto-Δ^{1,4}-steroids of the general formula I 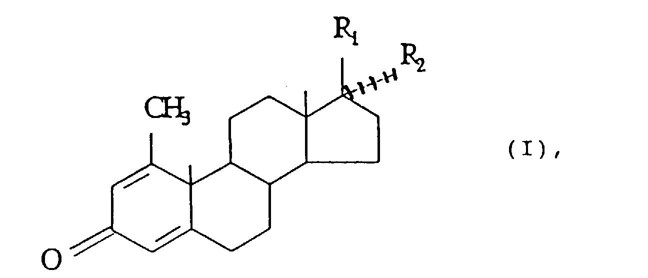 wherein
R₁ and R₂ together represent an oxo group or an alkylenedioxy group having from 2 to 6 carbon atoms, or wherein R₁ represents an acyloxy group having up to 8 carbon atoms or an alkyl radical having a maximum of 10 carbon atoms and
R₂ denotes a hydrogen atom,
characterised in that a 3-keto-Δ^{1,4}-steroid of the general formula II 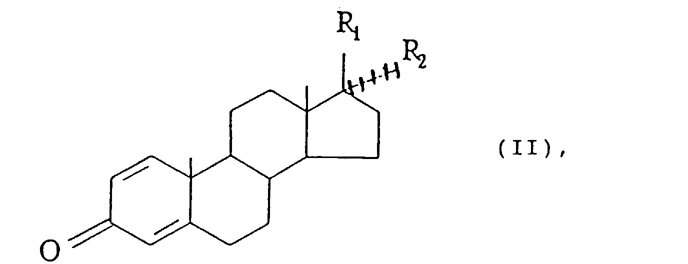 wherein R₁ and R₂ have the meanings given above, is reacted in an inert solvent, in the presence of a nickel salt soluble therein, with an organometallic compound of the formula III, IV or V
CH₃- Zn- X (III), 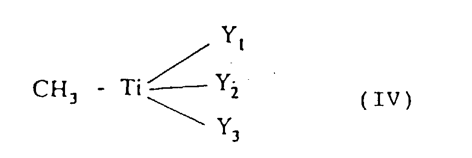
or 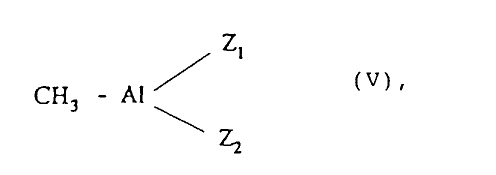 wherein
X represents a methyl group, an alkoxy group having a maximum of 6 carbon atoms, or a phenoxy radical optionally substituted by halogen atoms, alkoxy groups having a maximum of 6 carbon atoms and/or by alkyl groups having a maximum of 6 carbon atoms,
and
Y₁, Y₂ and Y₃ and also Z₁ and Z₂ are identical or different and have the same meanings as X,
at a temperature of from 40 to 80°C in one reaction step, the 1-methyl group being introduced while the double bond is re-formed simultaneously.

## Revendications

1. Procédé pour la préparation des 1-méthyl-3-cétoΔ^{1,4}-stéroïdes de formule générale I 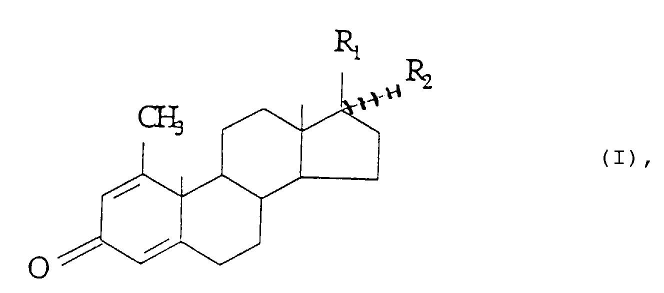 dans laquelle
R¹ et R² ensemble représentent un groupe oxo ou un groupe alkylènedioxy avec 2 à 6 atomes de carbone ou dans laquelle
R¹ représente un groupe acyloxy avec jusqu'à 8 atomes de carbone ou un radical alkyle avec au maximum 10 atomes de carbone et
R² représente un atome d'hydrogène,
caractérisé en ce que l'on prépare à partir de 3-céto-Δ^{1,4}-stéroides de formule générale II ci-dessous 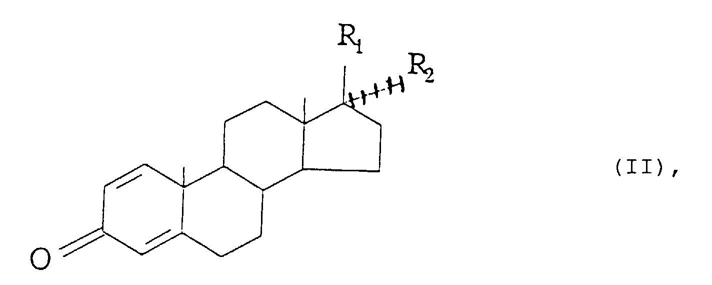 en faisant réagir, dans un solvant inerte, en présence d'un sel de nickel soluble dans ce solvant, avec un composé organométallique de formule III, IV ou V
CH₃-Zn-X (III), 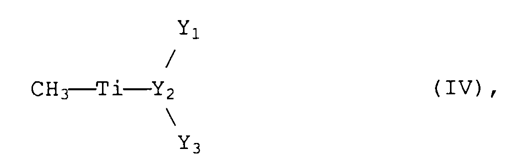 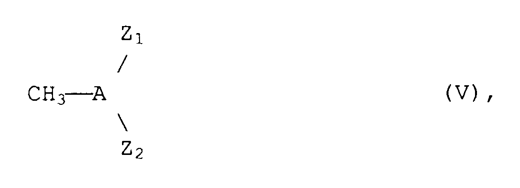
dans lesquelles
X représente un groupe méthyle, un groupe alcoxy avec au maximum 6 atomes de carbone ou un radical phényle éventuellement substitué par des atomes d'halogènes, des groupes alcoxy avec au maximum 6 atomes de carbone et/ou des groupes alkyle avec au maximum 6 atomes de carbone,
et
Y₁, Y₂ et Y₃ ainsi que Z₁ et Z₂ sont identiques ou différents et ont la même signification que X, à une température comprise entre 40 à 80 °C, dans une seule étape réactionnelle, en introduisant le groupe 1-méthyle en rétablissant simultanément la double liaison.
